# EUROPEAN PATENT APPLICATION

(11) **EP 4 092 116 A1**
(43) Date of publication of application: **23.11.2022**
(21) Application number: 21741220.4
(22) Date of filing: 13.01.2021
(51) Int. Cl.: C12N 9/80, C12N 9/86, C12N 15/55, A61K 38/50, A61P 19/06, A23L 33/10, A23L 33/13, A23L 33/18, A23L 33/175

(54) **XANTHINE AMIDE HYDROLASE AND USE THEREOF**

(30) Priority: 14.01.2020 CN 202010036500; 14.01.2020 CN 202010036519
(71) Applicant: Tianjin University, Tianjin 300072 (CN)
(72) Inventor: ZHANG, Yan, Tianjin 300072 (CN); LI, Peishan, Tianjin 300072 (CN); TONG, Yang, Tianjin 300072 (CN); HU, Yiling, Tianjin 300072 (CN); LIU, Dazhi, Tianjin 300072 (CN)
(74) Representative: Ipside
(86) International application number: PCT/CN2021/071501
(87) International publication number: WO 2021/143725

(57) **Abstract**

Provided are a xanthine amide hydrolase and the use thereof, particularly the use thereof for treating gout.

## Description

### Cross-references to related applications

This application claims the priority of Chinese Patent Application No. 202010036500.6 and No. 202010036519.0 filed on January 14, 2020, the entire contents of which are incorporated herein by reference in their entirety.

### Technical field

This application generally relates to the field of biomedicine technology; specifically, this application provides a new purine degradation pathway, enzymes participating in the pathway, and their usages, especially in the treatment of gout.

### Background technique

The absorption of purines mainly occurs in the intestine, which is an anaerobic environment. Gout is a type of arthritis caused by abnormal purine metabolism. It is characterized by recurring red, tender, burning, and swollen joints. The pain usually occurs quickly, reaching maximum intensity in less than 12 hours. The cause of gout is the increase in the level of uric acid in the body, which causes the deposition of urate in the joints and kidneys, and uric acid is a product of purine metabolism.

The existing drugs for gout include colchicine, allopurinol, febuxostat and other non-steroidal anti-inflammatory drugs and glucocorticoids. Colchicine inhibits the chemotaxis, adhesion and phagocytosis of neutrophils, so as to control local pain, swelling and inflammation in the joints. Allopurinol and febuxostat are selective xanthine oxidase inhibitors that can treat gout by reducing the blood urate concentration, but the above drugs have relatively large side effects.

The development and application of new drugs for the prevention, intervention and/or treatment of gout are needed in this field.

### Summary of the invention

In the first aspect, the present application provides a polypeptide comprising the amino acid sequence shown in SEQ ID NO:1 or a functional variant thereof, wherein the functional variant has xanthine amide hydrolase activity.

In some embodiments of the first aspect, wherein the polypeptide has a catalytic site defined as follows in spatial conformation:
The catalytic site comprises amino acid residues H59, H61, K151, H186, H242, and D316 that are close to each other in spatial conformation and refer to SEQ ID NO:1.

In some embodiments of the first aspect, the catalytic site further comprises a divalent metal ion.

In some embodiments of the first aspect, the polypeptide further comprises a binding site defined as follows in spatial conformation:
The binding site comprises amino acid residues I288, A289, P338 and G339 of SEQ ID NO:1 that are close to each other in spatial conformation.

In some embodiments of the first aspect, wherein the catalytic site is not more than 5 angstroms away from the binding site

In some embodiments of the first aspect, the functional variant is a natural isozyme of the amino acid sequence shown in SEQ ID NO:1.

In some embodiments of the first aspect, wherein the functional variant is the insertion, substitution and/or deletion of one or more amino acids on the basis of the amino acid sequence shown in SEQ ID NO:1 or its natural isozyme.

In some embodiments of the first aspect, wherein the insertion, substitution and/or deletion does not occur at the catalytic site and/or binding site.

In the second aspect, the present application provides a nucleic acid molecule that encodes the polypeptide described in the first aspect.

In the third aspect, the present application provides an expression cassette, which contains the nucleic acid molecule described in the second aspect.

In the fourth aspect, the present application provides an expression vector, which comprises the nucleic acid molecule described in the second aspect or the expression cassette described in the third aspect.

In the fifth aspect, the present application provides a host cell, which comprises the nucleic acid molecule described in the second aspect, the expression cassette described in the third aspect, or the expression vector described in the fourth aspect.

In some embodiments of the fifth aspect, the host cell can express and produce: a polypeptide comprising the amino acid sequence shown in SEQ ID NO:1 or a functional variant thereof, wherein the functional variant has xanthine amide hydrolysis enzyme activity.

In some embodiments of the fifth aspect, wherein the polypeptide has a catalytic site defined as follows in spatial conformation:

The catalytic site comprises amino acid residues H59, H61, K151, H186, H242, and D316 that are close to each other in spatial conformation and refer to SEQ ID NO:1.

In some embodiments of the fifth aspect, the catalytic site further comprises a divalent metal ion.

In some embodiments of the fifth aspect, the host cell is a eukaryotic cell or a prokaryotic cell.

In some embodiments of the fifth aspect, the eukaryotic cell is a yeast cell.

In some embodiments of the fifth aspect, the prokaryotic cell is selected from the group consisting of *Escherichia, Lactobacillus, Bifidobacterium, Bacteroides* and *Firmicutes*

In the sixth aspect, the present application provides a pharmaceutical composition or health food, which comprises the polypeptide described in the first aspect, the nucleic acid molecule described in the second aspect, the expression cassette described in the third aspect, and the expression vector described in the fourth aspect or the host cell and pharmaceutically acceptable carrier or excipient described in the fifth aspect.

In some embodiments of the sixth aspect, the pharmaceutical composition or health food is used for the prevention and/or intervention and/or treatment of gout.

In the seventh aspect, the present application provides the polypeptide described in the first aspect, the nucleic acid molecule described in the second aspect, the expression cassette described in the third aspect, the expression vector described in the fourth aspect, and the host cell described in the fifth aspect, or the pharmaceutical composition or health food in degrading purinesin the sixth aspect.

In some embodiments of the seventh aspect, the degradation of purines occurs in vitro.

In the eighth aspect, this application provides the polypeptide described in the first aspect, the nucleic acid molecule described in the second aspect, the expression cassette described in the third aspect, the expression vector described in the fourth aspect, and the host cell described in the fifth aspect, or the pharmaceutical composition or health food in the preparation of a medicament for the prevention, intervention and/or treatment of goutin the sixth aspect.

In the ninth aspect, this application provides a method for preventing, intervening and/or treating gout, which comprises giving toan individual in need the polypeptide of the first aspect, the nucleic acid molecule of the second aspect, and the expression cassette of the third aspect, the expression vector according to the fourth aspect, the host cell according to the fifth aspect, or the pharmaceutical composition or health food according to the sixth aspect.

### Description of the drawings

Figure 1 shows the human body's metabolic pathways that degrade various purines and the EC numbers of enzymes that catalyze each step of the reaction.
Figure 2 shows the metabolic reaction of xanthine oxidase to degrade xanthine to produce uric acid and its catalytic mechanism involving complex Mo-containing prosthetic groups.
Figure 3 shows the mechanism of allopurinol in the treatment of gout by inhibiting xanthine oxidase to prevent the production of uric acid.
Figure 4 shows the reaction formula (1) and the entire metabolic pathway for xanthine amide hydrolase to degrade xanthine under anaerobic conditions.
Figure 5 shows the electrophoresis results of the purified xanthine amide hydrolase, where lane M represents the molecular weight indicator, lane T is the bacterial lysate expressing xanthine amide hydrolase, and lane U represents the bacterial lysate after the Co affinity column, Lanes E1, E2, and E3 represent 1, 2 and 4 µg xanthine amide hydrolase purified and eluted by a cobalt affinity column, respectively.
Figure 6 shows the LC-MS (liquid mass spectrometry) test results of the enzymatic activity of xanthine amide hydrolase catalyzing the hydrolysis and opening of xanthine, where A is the LC-MS test results of the negative control without enzyme, and B is the LC-MS detection results of thetest group.
Figure 7 is a diagram of the active center of the xanthine amide hydrolase structure obtained by computer simulation by the PHYRE2 server (PHYRE2Protein Fold Recognition Server). The catalytic site contains fourhistidines, onecarboxylated lysine,one aspartic acid and two divalent metal ions (such as Zn²⁺ and/or Mn²⁺)that are close to each other in spatial conformation; the binding site contains one isoleucine,onealanine, one proline and one glycine residuethat are close to each other in spatial conformation and interact with xanthine.
Figure 8 is a phylogenetic tree diagram of all xanthine amide hydrolase enzymes in UniRef50_Q3AEA1. Gray markers are selected strains from each tree branch to represent *Bacillus firmus, Clostridium cylindrosporum* DSM 605, *Clostridium purinilyticum, Carbydothermus hydrogenoformans* strain ATCC BAA- 161/DSM 6008/Z-2901 and *Paenibacillus donghaensis,* the amino acid sequences of xanthine amide hydrolase encoded by them are A0A366K523, A0A0J8G334, A0A0L0W692, Q3AEA1 and A0A2Z2KEH1, respectively, and compared as in Figure 9.

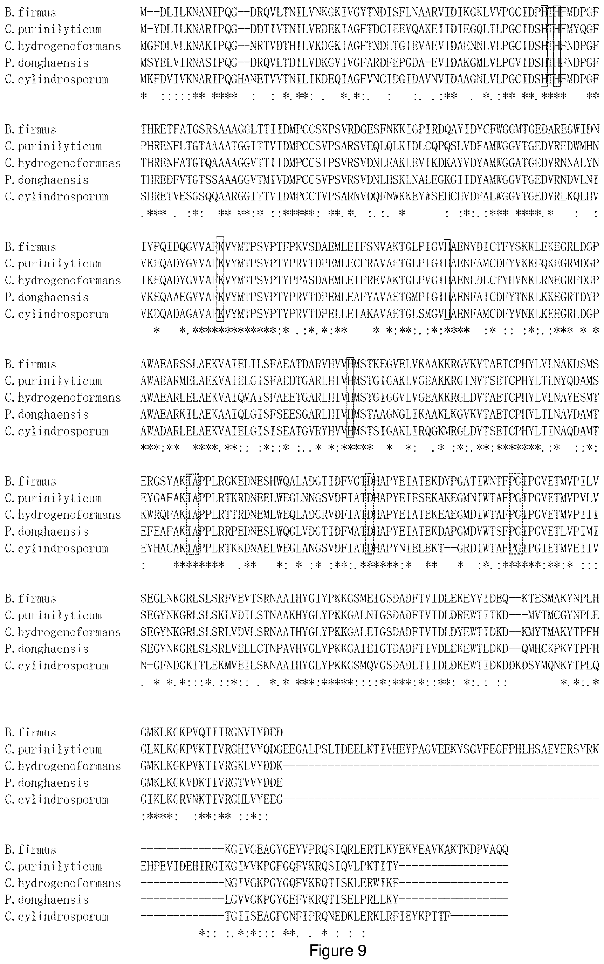

Figure 9 shows the xanthine amide hydrolase sequence comparison results of A0A366K523, A0A0J8G334, A0A0L0W692, Q3AEA1 and A0A2Z2KEH1 encoded by five different species of *Bacillus firmus, Clostridium cylindrosporum* DSM 605, *Clostridium purinilyticum,Carbydothermus hydrogenoformans* strain ATCC BAA-161/DSM 6008/ Z-2901 and *Paenibacillus donghaensis,* wherein the catalytic site contains two divalent metals (such as Zn²⁺ or Mn²⁺, etc.) and four histidine (H), one lysine (K) and one aspartic acid (D) residues (solid box) coordinated with the gray background; the binding site contains one isoleucine (I), one alanine (A), one proline (P) and one glycine residues (G)that binds to xanthine with gray background.

### Sequence description

SEQ ID NO: 1 shows the amino acid sequence of the xanthine amide hydrolase expressed by *Bacillus firmus*(CGMCC1.2010).
SEQ ID NO: 2 shows the nucleotide sequence of the upstream primer used for PCR amplification of the nucleic acid molecule encoding xanthine amide hydrolase.
SEQ ID NO: 3 shows the nucleotide sequence of the downstream primer used for PCR amplification of the nucleic acid molecule encoding xanthine amide hydrolase.
SEQ ID NO: 4 shows the amino acid sequence of the xanthine amide hydrolase expressed by *Clostridium purinilytica* with the accession number A0A0L0W692.
SEQ ID NO: 5 shows the amino acid sequence of the xanthine amide hydrolase with the accession number A0A364K317 expressed by *Thermoflavimicrobium sp.* FBKL4.011.
SEQ ID NO: 6 shows the amino acid sequence of the xanthine amide hydrolase expressed by the salt-tolerant marine filamentous bacteria *(Marininema halotolerans)* with the accession number A0A1I6SUY8.
SEQ ID NO: 7 shows the amino acid sequence of the xanthine amide hydrolase expressed by *Clostridiaceae bacterium* with the accession number A0A3D2NSM2.
SEQ ID NO: 8 shows the amino acid sequence of the xanthine amide hydrolase with the accession number E5WNF6 expressed by *Bacillus sp.*2_A_57_CT2.
SEQ ID NO: 9 shows the amino acid sequence of the xanthine amide hydrolase expressed by *Paenibacillus* sp.FSL H8-0259 with the accession number A0A1R1CK18.
SEQ ID NO: 10 shows the amino acid sequence of the xanthine amide hydrolase expressed by *Thermoflavimicrobium dichotomicum* with the accession number A0A1I3U261.
SEQ ID NO: 11 shows the amino acid sequence of the xanthine amide hydrolase expressed by *Fictibacillus enclensis* with the accession number A0A0V8JCN2.
SEQ ID NO: 12 shows the amino acid sequence of the xanthine amide hydrolase expressed by the marine filamentous bacteria *(Marininema mesophilum)* with the accession number A0A1H2QVP9.
SEQ ID NO: 13 shows the amino acid sequence of the xanthine amide hydrolase expressed by *Paenibacillus* sp.FSL R5-0912 with the accession number A0A089K5P4.
SEQ ID NO: 14 shows the amino acid sequence of the xanthine amide hydrolase expressed by *Paenibacillus typhae* with the accession number A0A1G9DGW2.
SEQ ID NO: 15 shows the amino acid sequence of the xanthine amide hydrolase expressed by *Tissierella praeacuta* DSM18095 with the accession number A0A1M4UHZ6.
SEQ ID NO: 16 shows the amino acid sequence of the xanthine amide hydrolase expressed by Bacillus cl95 *(Bacillus sp.* cl95) with the accession number A0A1I6C7J4.
SEQ ID NO: 17 shows the amino acid sequence of the xanthine amide hydrolase expressed by *Bradyrhizobium japonicum* with the accession number A0A0A3XEE6.
SEQ ID NO: 18 shows the amino acid sequence of the xanthine amide hydrolase expressed by *Bacillus firmus* with the accession number A0A380XTT6.
SEQ ID NO: 19 shows the amino acid sequence of the xanthine amide hydrolase expressed by *Acidaminobacter hydrogenoformans*DSM 2784 with the accession number A0A1G5S5P6.
SEQ ID NO: 20 shows the amino acid sequence of the xanthine amide hydrolase expressed by *Caloranaerobacter sp.* TR13 with the accession number A0A0P8Z9T7.
SEQ ID NO: 21 shows the amino acid sequence of xanthine amide hydrolase expressed by *Tissierella sp.* P1 with the accession number A0A265Q2B2.
SEQ ID NO: 22 shows the amino acid sequence of the xanthine amide hydrolase expressed by *Bacillus sp.* 3-2-2 with the accession number A0A429Y566.
SEQ ID NO: 23 shows the amino acid sequence of the xanthine amide hydrolase expressed by *Paenibacillus donghaensis* with the accession number A0A2Z2KEH1.
SEQ ID NO: 24 shows the amino acid sequence of the xanthine amide hydrolase with the accession number K0AWA5 expressed by *Gottschalkia acidurici* (strain ATCC7906/DSM 604/BCRC 14475/CIP 104303/NCIMB 10678/9a).
SEQ ID NO: 25 shows the amino acid sequence of the xanthine amide hydrolase expressed by *Bacillus fortis* with the accession number A0A443IKX3.
SEQ ID NO: 26 shows the amino acid sequence of the xanthine amide hydrolase expressed by *Bacillus oceanisediminis* with the accession number A0A2V2ZNB0.
SEQ ID NO: 27 shows the amino acid sequence of the xanthine amide hydrolase expressed by *Virgibacillus profundi* with the accession number A0A2A2IEE5.
SEQ ID NO: 28 shows the amino acid sequence of the xanthine amide hydrolase expressed by *Paenibacillus sp.* FSL R7-0331 with the accession number A0A089MDW3.
SEQ ID NO: 29 shows the amino acid sequence of the xanthine amide hydrolase expressed by *Bacillus sp.* FJAT-21945) with the accession number A0A0M0X8C9.
SEQ ID NO: 30 shows the amino acid sequence of xanthine amide hydrolase expressed by *Tissierella praeacuta* with the accession number A0A3F3S6I2.
SEQ ID NO: 31 shows the amino acid sequence of xanthine amide hydrolase expressed by *Bacillus oceanisediminis* with the accession number A0A1S1YDG4.
SEQ ID NO: 32 shows the amino acid sequence of the xanthine amide hydrolase expressed by *Bacillus sp.* OV194 with the accession number A0A1I1X526.
SEQ ID NO: 33 shows the amino acid sequence of the xanthine amide hydrolase expressed by *Anaeromicrobiumsediminis* with the accession number A0A267MJF0.
SEQ ID NO: 34 shows the amino acid sequence of the xanthine amide hydrolase whose accession number is A0A1M5RDL0 expressed by *Thermosyntropha lipolytica* DSM 11003.
SEQ ID NO: 35 shows the amino acid sequence of the xanthine amide hydrolase expressed by *Alkaliphilus peptidifermentans* DSM 18978 with the accession number A0A1G5KXH0.
SEQ ID NO: 36 shows the amino acid sequence of the xanthine amide hydrolase expressed by *Aneurinibacillus migulanus* with the accession number A0A1G8Q7H9.
SEQ ID NO: 37 shows the amino acid sequence of the xanthine amide hydrolase expressed by *Paenibacillus sp.* IHB B 3415 with the accession number A0A0B2F3Z2.
SEQ ID NO: 38 shows the amino acid sequence of the xanthine amide hydrolase expressed by *Marinisporobacter balticus* with the accession number A0A4R2KME8.
SEQ ID NO: 39 shows the amino acid sequence of the xanthine amide hydrolase expressed by *Bacillus terrae* with the accession number A0A429X0W8.
SEQ ID NO: 40 shows the amino acid sequence of the xanthine amide hydrolase expressed by *Tindallia californiensis* with the accession number A0A1H3Q6T8.
SEQ ID NO: 41 shows the amino acid sequence of the xanthine amide hydrolase with the accession number K0B360 expressed by *Clostridium acidurici(strain* ATCC7906/DSM 604/BCRC 14475/CIP 104303/NCIMB 10678/9a).
SEQ ID NO: 42 shows the amino acid sequence of the xanthine amide hydrolase expressed by *Romboutsia lituseburensis* DSM 797 with the accession number A0A1G9M635.
SEQ ID NO: 43 shows the amino acid sequence of the xanthine amide hydrolase expressed by *Paraclostridium bifermentans* ATCC 19299 with the accession number T4V5T0.
SEQ ID NO: 44 shows the amino acid sequence of the xanthine amide hydrolase expressed by *Bacilluspraedii* with the accession number A0A4R1ATL6.
SEQ ID NO: 45 shows the amino acid sequence of the xanthine amide hydrolase expressed by *Carbydothermus islandicus* with the accession number A0A1L8D5S0.
SEQ ID NO: 46 shows the amino acid sequence of the xanthine amide hydrolase expressed by *Caloramator australicus* RC3 with the accession number I7KTT3.
SEQ ID NO: 47 shows the amino acid sequence of the xanthine amide hydrolase with the accession number T4VRB8 expressed by *Paraclostridium bifermentans* ATCC 638.
SEQ ID NO: 48 shows the amino acid sequence of the xanthine amide hydrolase expressed by *Tepidimicrobium xylanilyticumwith* the accession number A0A1H2RLU1.
SEQ ID NO: 49 shows the amino acid sequence of the xanthine amide hydrolase expressed by *Aneurinibacillus migulanus* with the accession number A0A0K2WJ73.
SEQ ID NO: 50 shows the amino acid sequence of the xanthine amide hydrolase expressed by *Bacillus bacterium* with the accession number A0A3D0EBN7.
SEQ ID NO: 51 shows the amino acid sequence of the xanthine amide hydrolase expressed by *Bacillusnotoginsengisoli* with the accession number A0A417YW08.
SEQ ID NO: 52 shows the amino acid sequence of the xanthine amide hydrolase expressed by *Bacillus oceanisediminis* 2691 with the accession number A0A160MBB4.
SEQ ID NO: 53 shows the amino acid sequence of the xanthine amide hydrolase expressed by *Paenibacillus* sp.FSL R7-0273 with the accession number A0A089LXU3.
SEQ ID NO: 54 shows the amino acid sequence of the xanthine amide hydrolase with the accession number M1ZGS5 expressed by *[Clostridium] ultunense Esp.*
SEQ ID NO: 55 shows the amino acid sequence of the xanthine amide hydrolase expressed by *Bacillus freudenreichii* with the accession number A0A448FF64.
SEQ ID NO: 56 shows the amino acid sequence of the xanthine amide hydrolase expressed by *Caloramator fervidus* with the accession number A0A1H5RUL9.
SEQ ID NO: 57 shows the amino acid sequence of xanthine amide hydrolase expressed by *Bacillus oceanisediminis* with the accession number A0A4R8GVS7.
SEQ ID NO: 58 shows the amino acid sequence of the xanthine amide hydrolase expressed by *Soehngenia saccharolytica* with the accession number A0A4T9ZWH0.
SEQ ID NO: 59 shows the amino acid sequence of the xanthine amide hydrolase with the accession number W7LB72 expressed by *Bacillus firmus* DS1.
SEQ ID NO: 60 shows the amino acid sequence of the xanthine amide hydrolase expressed by *Thermotalea metallivorans* with the accession number A0A140L3I5.
SEQ ID NO: 61 shows the amino acid sequence of the xanthine amide hydrolase expressed by *Ornithinibacillus halophilus* with the accession number A0A1M5FNK3.
SEQ ID NO: 62 shows the amino acid sequence of the xanthine amide hydrolase whose accession number is A0A1M5LQE8 expressed by *Thermosyntropha lipolytica*DSM 11003.
SEQ ID NO: 63 shows the amino acid sequence of the xanthine amide hydrolase expressed by *Fictibacillus sp.* S7 with the accession number A0A4Q2HRQ1.
SEQ ID NO: 64 shows the amino acid sequence of the xanthine amide hydrolase expressed by *Bacillus mesonae* with the accession number A0A3Q9QZ31.
SEQ ID NO: 65 shows the amino acid sequence of the xanthine amide hydrolase expressed by *Clostridium purinilytica* with the accession number A0A0L0W688.
SEQ ID NO: 66 shows the amino acid sequence of the xanthine amide hydrolase expressed by *Tindallia magadiensis* with the accession number A0A1I3ETA9.
SEQ ID NO: 67 shows the amino acid sequence of the xanthine amide hydrolase expressed by *Paenibacillus borealis* with the accession number A0A089LHF8.
SEQ ID NO: 68 shows the amino acid sequence of the xanthine amide hydrolase expressed by *Paraclostridium benzoelyticum* with the accession number A0A0M3DN30.
SEQ ID NO: 69 shows the amino acid sequence of the xanthine amide hydrolase expressed by *Bacillus solani* with the accession number A0A0Q3QMR7.
SEQ ID NO: 70 shows the amino acid sequence of the xanthine amide hydrolase expressed by *Thermohalobacterberrensis* with the accession number A0A419T2I0.
SEQ ID NO: 71 shows the amino acid sequence of the xanthine amide hydrolase expressed by *Acinetobacter sp.* RIT592 with the accession number A0A369PBX6.
SEQ ID NO: 72 shows the amino acid sequence of the xanthine amide hydrolase expressed by *Maledivibacter halophilus* with the accession number A0A1T5JUM9.
SEQ ID NO: 73 shows the amino acid sequence of the xanthine amide hydrolase expressed by *Tissierella creatinini* with the accession number A0A4T9WHZ3.
SEQ ID NO: 74 shows the amino acid sequence of the xanthine amide hydrolase expressed by the *compost metagenome* with the accession number A0A3R1HSK2.
SEQ ID NO: 75 shows the amino acid sequence of the xanthine amide hydrolase expressed by *Natronincola peptidivorans* with the accession number A0A1I0F3P2.
SEQ ID NO: 76 shows the amino acid sequence of the xanthine amide hydrolase expressed by *Bacillus firmus* with the accession number A0A0J5YTU9.
SEQ ID NO: 77 shows the amino acid sequence of the xanthine amide hydrolase expressed by *Anaerovirgula multivorans* with the accession number A0A239FV01.
SEQ ID NO: 78 shows the amino acid sequence of the xanthine amide hydrolase with the accession number Q3AEA1 expressed by *Carbydothermus hydrogenoformans* (strain ATCC BAA-161/DSM 6008/Z-2901).
SEQ ID NO: 79 shows the amino acid sequence of xanthine amide hydrolase expressed by *Sporanaerobacter acetigenes* DSM 13106 with the accession number A0A1M5YST8.
SEQ ID NO: 80 shows the amino acid sequence of the xanthine amide hydrolase expressed by *Proteiniborus sp.* DW1 with the accession number A0A1M4MA63.
SEQ ID NO: 81 shows the amino acid sequence of the xanthine amide hydrolase expressed by *Bacillus* sp.7894-2 with the accession number A0A268IWM5.
SEQ ID NO: 82 shows the amino acid sequence of the xanthine amide hydrolase with the accession number A0A1M4PLJ1 expressed by *[Clostridium] ultunense Esp.*
SEQ ID NO: 83 shows the amino acid sequence of the xanthine amide hydrolase expressed by *Virgibacillus indicus* with the accession number A0A265N7L4.
SEQ ID NO: 84 shows the amino acid sequence of the xanthine amide hydrolase expressed by *Paenibacillus sp.* NFR01 with the accession number A0A1I0KAI3.
SEQ ID NO: 85 shows the amino acid sequence of the xanthine amide hydrolase expressed by *Andreesenia angus* with the accession number A0A1S1V3Q5.
SEQ ID NO: 86 shows the amino acid sequence of the xanthine amide hydrolase expressed by *Paenibacillus sp.* DMB5with the accession number A0A117T0P2.
SEQ ID NO: 87 shows the amino acid sequence of the xanthine amide hydrolase expressed by *Paludifilum halophilum* with the accession number A0A235B9X8.
SEQ ID NO: 88 shows the amino acid sequence of the xanthine amide hydrolase expressed by *Proteiniborus ethanoligenes* with the accession number A0A1H3NJW1.
SEQ ID NO: 89 shows the amino acid sequence of the xanthine amide hydrolase expressed by *Alkaliphilus metalliredigens* (strain QYMF) with the accession number A6TWT7.
SEQ ID NO: 90 shows the amino acid sequence of the xanthine amide hydrolase expressed by *Fictibacillussolisalsi* with the accession number A0A1G9U6Z5.
SEQ ID NO: 91 shows the amino acid sequence of the xanthine amide hydrolase expressed by *Sporosarcina globispora* with the accession number A0A0M0GGH4.
SEQ ID NO: 92 shows the amino acid sequence of the xanthine amide hydrolase with the accession number A0A366K523 expressed by *Bacillus firmus.*
SEQ ID NO: 93 shows the amino acid sequence of the xanthine amide hydrolase expressed by *Paenibacillus* sp.FSL R5-0490 with the accession number A0A1R1FFH4.
SEQ ID NO: 94 shows the amino acid sequence of the xanthine amide hydrolase expressed by *Alkaliphilus sp.* with the accession number A0A2G2MLE4.
SEQ ID NO: 95 shows the amino acid sequence of the xanthine amide hydrolase with the accession number A8MLA7 expressed by anaerobic arsenic-reducing bacteria *(Alkaliphilus oremlandii,* strain OhILAs).
SEQ ID NO: 96 shows the amino acid sequence of the xanthine amide hydrolase expressed by *Caloramator mitchellensis* with the accession number A0A0R3JVG6.
SEQ ID NO: 97 shows the amino acid sequence of the xanthine amide hydrolase expressed by *Clostridium cylindrosporum*DSM 605 with the accession number A0A0J8G334.
SEQ ID NO: 98 shows the amino acid sequence of the xanthine amide hydrolase expressed by *Ammoniphilus sp.* CFH 90114 with the accession number A0A4Q1SWC2.
SEQ ID NO: 99 shows the amino acid sequence of the xanthine amide hydrolase expressed by *Carbydothermus pertinax* with the accession number A0A1L8CSM0.
SEQ ID NO: 100 shows the amino acid sequence of the xanthine amide hydrolase expressed by *Soehngenia sp.* 1933P with the accession number A0A4Z0D783.
SEQ ID NO: 101 shows the amino acid sequence of the xanthine amide hydrolase expressed by *Soehngenia saccharolytica* with the accession number A0A4T9ZOT2.
SEQ ID NO: 102 shows the amino acid sequence of the xanthine amide hydrolase expressed by *Natribacillus halophilus* with the accession number A0A1G8N2H8.
SEQ ID NO: 103 shows the amino acid sequence of the xanthine amide hydrolase expressed by the *compost metagenome* with the accession number A0A403WBU6.
SEQ ID NO: 104 shows the amino acid sequence of the xanthine amide hydrolase with the accession number A0A3R4A6V9 expressed by the *compost metagenome.*

### Detailed description of the invention

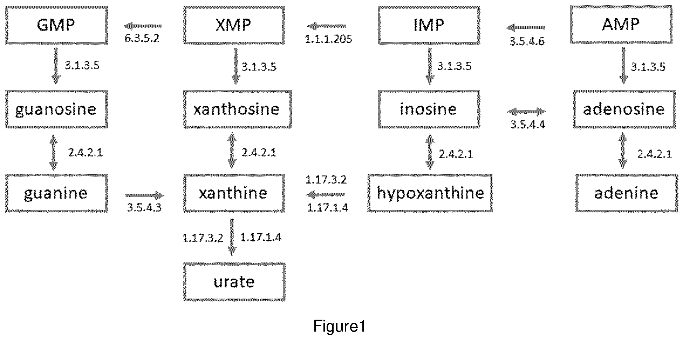

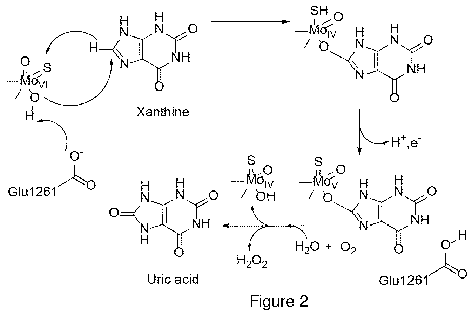

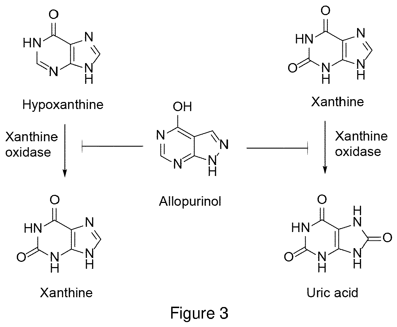

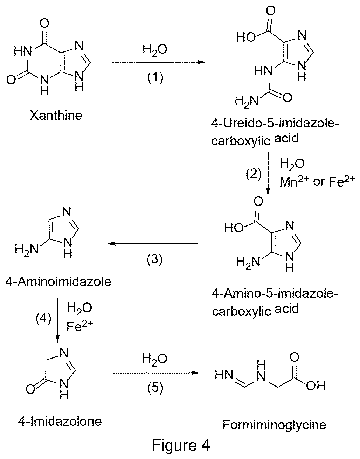

Figure 1 shows the metabolic pathways of various purines in the human body. The final product of various purine metabolism is urate, and the deposition of urate in joints and kidneys is the cause of gout. Figure 2 shows the process by which the aerobic protein xanthine oxidase catalyzes the degradation of xanthine to produce uric acid. The gout drug allopurinol inhibits the production of uric acid by inhibiting xanthine oxidase, thereby treating gout (Figure 3). G.D. VOGELS et al. (Degradation of Purines and Pyrimidines by Microorganisms, Bacteriological Reviews, June 1976, Vol. 40, No. 2, p. 403-468) reported that *Clostridium cylindrosporum* can degrade xanthine to iminomethylglycineunder anaerobic conditions. Figure 4 shows the reaction formula of xanthine amide hydrolase to degrade xanthine under anaerobic conditions. The inventor of the present application realizes that purine degradation under anaerobic conditions is of great significance, because the human intestinal tract is an anaerobic environment. If it can precede the absorption of purines in the intestinal tract and degrade purines in the intestinal tract, this is a new idea for prevention, intervention, alleviation and/or treatment of gout. The inventor of the present application successfully identified xanthine amide hydrolase that degrades xanthine under anaerobic conditions after doing a lot of work using bioinformatics methods.

Unless otherwise specified, the terms used in this application have the meanings commonly understood by those skilled in the field.

### Definition

Unless otherwise indicated, nucleic acids are written from left to right in the 5'to 3'direction, respectively; amino acid sequences are written from left to right in the amino to carboxy direction, respectively. The number range includes the number that defines the range. Amino acids can be represented herein by their commonly known three-letter symbols or the one-letter symbols recommended by the IUPAC-IUB Biochemical Nomenclature Committee. Likewise, the commonly accepted one-letter codes can be used to represent nucleotides. Refer to the specification as a whole to more fully define the terms defined above.

"Polypeptide" and "protein" in this application are used interchangeably herein and refer to polymers of amino acid residues and their variants and synthetic and naturally-occurring analogs. Therefore, these terms apply to naturally-occurring amino acid polymers and their naturally-occurring chemical derivatives, and to non-naturally-occurring amino acids in which one or more amino acid residues are synthetic (such as chemical analogs of the corresponding naturally-occurring amino acids) amino acid polymer. Such derivatives include, for example, post-translational modifications and degradation products, including phosphorylated, glycosylated, oxidized, isomerized, carboxylated, and deaminated variants of polypeptide fragments.

The term "enzyme active center" as used herein refers to the part of the enzyme molecule that can directly bind to the substrate molecule and catalyze the chemical reaction of the substrate, and this part becomes the active center of the enzyme. It is generally believed that the active center is mainly composed of two functional sites: the first is the catalytic site, where the bond of the substrate is broken or a new bond is formed to cause certain chemical changes; the second is the binding site, which is the substrate of the enzyme,the substance binds to the enzyme molecule by this site. The functional part is composed of a few amino acid residues or some groups on these residues that are relatively close in the three-dimensional structure of the enzyme molecule. They may be far apart in the primary structure, or even located on different peptide chains, but close to each other in spatial conformation through the winding and folding of the peptide chain; for enzymes that require coenzymes, coenzyme molecules (such as metal ions Zn²⁺ and/or Mn²⁺) or a certain part of the structure of the coenzyme molecule is also a function part of the site.

The term "amino acid" as used herein refers to a compound in which a hydrogen atom on a carbon atom of a carboxylic acid is replaced by an amino group, and the amino acid molecule contains two functional groups: an amino group and a carboxyl group. It includes naturally occurring and non-naturally occurring amino acids as well as amino acid analogs and mimetics. Naturally-occurring amino acids include 20 kinds of (L)-amino acids used in protein biosynthesis and other amino acids, such as 4-hydroxyproline, hydroxylysine, carboxylated lysine, catenin, isocatenin, high cysteine, citrulline and ornithine. Non-naturally occurring amino acids include, for example, (D)-amino acids, norleucine, norvaline, p-fluorophenylalanine, ethylthioine, etc., which are known to those skilled in the art. Amino acid analogs include modified forms of naturally occurring and non-naturally occurring amino acids. Such modifications may include, for example, substitution of chemical groups and moieties on amino acids, or derivatization of amino acids. Amino acid mimetics include, for example, organic structures that exhibit functionally similar properties, such as the charge and charge space properties of amino acids. For example, an organic structure that mimics arginine (Arg or R) has a positively charged moiety that is located in a similar molecular space and has the same degree of mobility as the e-amino group of the side chain of a naturally occurring Arg amino acid. Mimics also include constrained structures to maintain optimal space and charge interactions of amino acids or amino acid functional groups. Those skilled in the art can determine what structures constitute functionally equivalent amino acid analogs and amino acid mimetics.

The term "isoenzyme" as used herein refers to enzymes that catalyze the same reaction in an organism but have different molecular structures.

The term "nucleic acid" as used herein refers to mRNA, RNA, cRNA, cDNA or DNA, including single-stranded and double-stranded forms of DNA. The term generally refers to a polymeric form of nucleotides of at least 10 bases in length, the nucleotides being ribonucleotides or deoxynucleotides or a modified form of any type of nucleotide.

As used herein, the term "encoding" when used in the context of a specific nucleic acid means that the nucleic acid contains the necessary information to direct the translation of the nucleotide sequence into a specific protein. The use of codons represents information about the encoded protein. A nucleic acid encoding a protein may comprise untranslated sequences (e.g., introns) located within the translation region of the nucleic acid or may lack such intervening untranslated sequences (e.g., as in cDNA).

As used herein, a "full-length sequence" related to a specific polynucleotide or the protein encoded by it refers to the entire nucleic acid sequence or the entire amino acid sequence having a natural (non-synthetic) endogenous sequence. The full-length polynucleotide encodes the full-length, catalytically active form of the specific protein.

The term "isolated" as used herein refers to a polypeptide or nucleic acid or a biologically active portion thereof, which is substantially or essentially free of those normally accompanied or reacted with the protein or nucleic acid as found in its naturally occurring environment components. Therefore, when recombinant technology is used to produce isolated polypeptides or nucleic acids, the isolated polypeptides or nucleic acids are substantially free of other cellular materials or culture media, or when the isolated polypeptides or nucleic acids are chemically synthesized, they are substantially free of chemical precursors or other chemicals.

The term "expression vector" as used herein is a recombinantly or synthetically produced nucleic acid construct that has a series of specific nucleic acid elements that allow specific nucleic acid to be transcribed in a host cell.

The term "host cell" as used herein refers to a cell that receives a foreign gene in transformation and transduction (infection). The host cell may be a eukaryotic cell such as a yeast cell or a prokaryotic cell such as *E. coli.*

### Specific implementation plan:

In the first aspect, the present application provides a polypeptide comprising the amino acid sequence shown in SEQ ID NO:1 or a functional variant thereof, wherein the functional variant has xanthine amide hydrolase activity.

In some embodiments of the first aspect, it has a catalytic site defined as follows in spatial conformation:
The catalytic site comprises amino acid residues H59, H61, K151, H186, H242, and D316 that are close to each other in spatial conformation and refer to SEQ ID NO:1.

In some embodiments of the first aspect, the catalytic site further comprises a divalent metal ion.

In some specific embodiments of the first aspect, the catalytic site further comprises two divalent metal ions, such as Zn²⁺ and/or Mn²⁺.

In some embodiments of the first aspect, wherein the polypeptide further comprises a binding site having the following definition in a spatial conformation:
The binding site comprises amino acid residues I288, A289, P338 and G339 of SEQ ID NO:1 that are close to each other in spatial conformation.

In some embodiments of the first aspect, the distance between the catalytic site and the binding site is no more than 5 angstroms

In some embodiments of the first aspect, wherein the functional variant is a natural isozyme of the amino acid sequence shown in SEQ ID NO:1.

In some embodiments of the first aspect, the natural isozyme is derived from: *Bacillus firmus, compost metagenome, Clostridium purinilyticum, Thermoflavimicrobium sp., Marininemahalotolerans, Clostridiaceae bacterium, Bacillus sp., Paenibacillus sp., Thermoflavimicrobium dichotomicum, Fictibacillusenclensis, Marininema mesophilum, Paenibacillus typhae, Tissierella praeacuta, Bradyrhizobium japonicum, Acidaminobacter hydrogenoformans, Caloranaerobacter sp., Tissierella sp., Paenibacillus donghaensis, Gottschalkia acidurici, Clostridium acidurici, Bacillus fortis, Bacillus oceanisediminis, Virgiedbacillus profundi, Anaeromicrobium sediminis, Thermosyntropha lipolytica, Alkaliphilus peptidifermentans, Aneurinibacillus migulanus, Bacillus bacterium, Marinisporobacter balticus, Bacillus terrae,* California *Tindallia californiensis, Romboutsia lituseburensis, Paraclostridium bifermentans, Bacillus praedii, Carbydothermus islandicus, Caloramator australicus, Paraclostridium bifermentans, Tepidimicrobium xylanilyticum, Bacillus notoginsengisoli, [Clostridium] ultunense Esp, Bacillus freudenreichii, Caloramator fervidus, Soehngenia saccharolytica, Thermotalea metallivorans, Ornithinibacillus halophilus, Fictibacillussp., Bacillus mesonae, Tindallia magadiensis, Paenibacillus borealis, Paraclostridium benzoelyticum, Bacillus solani, Thermohalobacter berrensis, Acinetobactersp., Maledivibacter halophilus, Tissierella creatinini, Natronincola peptidivorans, Anaerovirgula multivorans, Carbydothermus hydrogenoformans, Sporanaerobacter acetigenes, Proteiniborus sp., Virgibacillus indicus, Andreesenia angusta, Paludifilum halophilum, Proteiniborus ethanoligenes, Alkaliphilus metalliredigens, Fictibacillus solisalsi, Sporosarcina globispora, Alkaliphilus sp., Alkaliphilus oremlandii, Caloramator mitchellensis, Clostridium cylindrosporum, Ammoniphilus sp., Carbydothermus pertinax, Soehngenia sp.* and *Natribacillus halophilus.*

In some specific embodiments of the first aspect, the natural isozyme comprises the amino acid sequence shown in any one of SEQ ID NO: 4-104.

In some specific embodiments of the first aspect, wherein the functional variant is the insertion, substitution and/or deletion of one or more amino acids on the basis of the amino acid sequence shown in SEQ ID NO:1 or its natural isozymes.

In some embodiments of the first aspect, the insertion, substitution and/or deletion does not occur at the catalytic site.

In some embodiments of the first aspect, the insertion, substitution and/or deletion does not occur at the binding site.

In some embodiments of the first aspect, the insertion, substitution and/or deletion does not occur at the catalytic site and the binding site.

In some embodiments of the first aspect, the number of amino acid insertions, substitutions and/or deletions is 1-30, preferably 1-20, more preferably 1-10, wherein the obtained functional variant is basically remaining the activity of xanthine amide hydrolase unchanged.

In some embodiments of the first aspect, the functional variant differs from the amino acid sequence shown in SEQ ID NO:1 by about 1, 2, 3, 4, 5, 6, 7, 8, 9, or 10 amino acids insertions, substitutions and/or deletions.

In some embodiments of the first aspect, the functional variant differs from the amino acid sequence shown in SEQ ID NO: 1 by about 1, 2, 3, 4 or 5 amino acid insertions, substitutions and/or deletions.

In some embodiments of the first aspect, the polypeptide is an isolated polypeptide.

In the second aspect, the present application provides a nucleic acid molecule that encodes the polypeptide described in the first aspect.

In some embodiments of the second aspect, the nucleic acid molecule of the present application comprises a nucleic acid that hybridizes to a nucleotide sequence encoding the polypeptide shown in any one of SEQ ID NO: 1 and SEQ ID NO: 4-104 under stringent conditions. A nucleotide sequence, or a nucleotide sequence that specifically hybridizes with any one of the polypeptides of SEQ ID NO: 1 and SEQ ID NO: 4-104 and is encoded with SEQ ID NO: 1 and SEQ ID NO: 4-104 the nucleic acid sequence composition of a polypeptide that is functionally equivalent to the polypeptide shown in any one of the items.

Those skilled in the art can routinely select stringent conditions for DNA hybridization. Generally longer probes require higher temperatures for proper annealing, while shorter probes require lower temperatures. Hybridization generally depends on the ability of denatured DNA to reanneal when the complementary strand is in an environment below its melting temperature. The higher the degree of homology between the probe and the hybridizable sequence, the higher the relative temperature that can be used. Therefore, a higher relative temperature tends to make the reaction conditions more stringent, while at a lower temperature, the stringency is lower. For a detailed description of the stringent conditions of the hybridization reaction, please refer to Ausubel et al., Current Protocols in Molecular Biology, Wiley Interscience Publishers, (1995).

In some embodiments of the second aspect, stringent conditions used for DNA hybridization include: 1) Low ionic strength and high temperature are used for washing, for example, 0.015M sodium chloride/0.0015M sodium citrate/0.1% sodium alkyl sulfate at 50°C; 2) Use formamide and other denaturants during hybridizationat 42°C, such as 50% (v/v) formamide plus 0.1% bovine serum albumin/0.1% Ficoll/0.1% polydiene pyrrolidone/50mM sodium phosphate buffer (pH 6.5), 750mM sodium chloride, 75mM sodium citrate; and or (3) hybridize overnight at 42°C, the hybridization solution contains 50% formamide, 5xSSC (0.75M sodium chloride, 0.075M citric acid sodium), 50mM sodium phosphate (pH 6.8), 0.1% sodium pyrophosphate, 5xDenhardt's solution, sonicated salmon sperm DNA (50mg/mL), 0.1% SDS and 10% dextran sulfate, then in 0.2xSSC (sodium chloride/sodium citrate) was washed at 42°C for 10 minutes, and then washed with high stringency at 55°C with 0.1×SSC containing EDTA. The moderately stringent conditions can be determined as described in Sambrook et al., Molecular Cloning: A Laboratory Manual, New York: Cold Spring Harbor Press, 1989. Moderately stringent conditions include the use of washing solutions and hybridization conditions (such as temperature, ionic strength, and SDS percentage) that are less stringent than those described above. For example, moderately stringent conditions include hybridization with at least about 16% v/v to at least about 30% v/v formamide and at least about 0.5 M to at least about 0.9 M salt at 42°C, and at least about 0.1 M to at least about 0.2M salt is washed at 55°C. Moderately stringent conditions can also include hybridization with 1% bovine serum albumin (BSA), 1mM EDTA, 0.5M NaHPO₄ (pH 7.2), 7% SDS at 65°C, and (i) 2xSSC, 0.1% SDS; or (ii) 0.5% BSA, 1mM EDTA, 40mM NaHPO₄ (pH 7.2), 5% SDS wash at 60-65°C. Professionals will adjust the temperature and ionic strength according to the length of the probe and other factors. The stringency of hybridizing nucleic acids depends on the length and degree of complementarity of the nucleic acid molecules, as well as other variables well known in the art. The greater the similarity or homology between two nucleotide sequences, the greater the Tm of nucleic acid hybrids containing these sequences. The relative stability of nucleic acid hybridization (corresponding to higher Tm) decreases in the following order: RNA:RNA, DNA:RNA, DNA:DNA. Preferably, the minimum length of a hybridizable nucleic acid is at least about 12 nucleotides, preferably at least about 16, more preferably at least about 24, and most preferably at least about 36 nucleotides.

The nucleic acid molecules of the present application can be combined with other DNA sequences, such as promoters, polyadenylation signals, other restriction sites, multiple cloning sites, other coding segments, etc., so that their total length can vary significantly. It is therefore considered that polynucleotide fragments of almost any length can be utilized; the total length is preferably limited by the ease of preparation and use in the intended recombinant DNA protocol.

Any one of a variety of mature technologies known and available in the art can be used to prepare, manipulate, and/or express polynucleotides and their fusions. For example, nucleic acid molecules encoding the polypeptides of the present application or functional variants thereof can be used in recombinant DNA molecules to direct the expression of the polypeptides in appropriate host cells. Due to the inherent degeneracy of the genetic code, other DNA sequences encoding substantially the same or functionally equivalent amino acid sequences can also be used in this application, and these sequences can be used to clone and express a given polypeptide.

In addition, methods known in the art can be used to modify the nucleic acid molecules of the present application, including but not limited to the cloning, processing, expression and/or activity modification of gene products.

In some embodiments of the second aspect, the nucleic acid molecule is produced by artificial synthesis, such as direct chemical synthesis or enzymatic synthesis.

In some embodiments of the second aspect, the nucleic acid molecule is produced by recombinant technology.

In some embodiments of the second aspect, the nucleic acid molecule is an isolated nucleic acid molecule.

In the third aspect, the present application provides an expression cassette, which contains the nucleic acid molecule described in the second aspect.

In some specific embodiments of the third aspect, the expression cassette may additionally include a 5'leader sequence, which can play a role in enhancing translation.

When preparing the expression cassette, various DNA fragments can be manipulated to provide the DNA sequence in the proper orientation and in the proper reading frame when appropriate. To achieve this goal, adaptors or linkers can be used to connect DNA fragments, or other operations can be involved to provide convenient restriction sites, remove excess DNA, remove restriction sites, and so on. For this purpose, it may involve in vitro mutagenesis, primer repair, restriction, annealing, and replacement, such as transition and transversion.

In the fourth aspect, the present application provides an expression vector, which comprises the nucleic acid molecule described in the second aspect or the expression cassette described in the third aspect.

In some embodiments of the fourth aspect, any suitable expression vector can be used in this application. For example, the expression vector can be any of pET28, pET14, HT-1N-TAG-2691, pRS416 and other vectors.

In some embodiments of the fourth aspect, the nucleic acid molecule encoding the polypeptide shown in any one of SEQ ID NO: 1 and SEQ ID NO: 4-104 is cloned into a recombinant vector to form a nucleic acid molecule containing the nucleic acid molecule described in the present application.

In some embodiments of the fourth aspect, the expression vector used to clone the polynucleotide is a plasmid vector.

In some embodiments of the fourth aspect, the above-mentioned expression vector further comprises a control sequence for regulating the expression of the nucleic acid molecule, wherein the nucleic acid molecule is operably linked to the control sequence.

The term "regulatory sequence" as used herein refers to a polynucleotide sequence required to achieve expression of a coding sequence linked to it. The nature of such regulatory sequences varies with the host organism. In prokaryotes, such regulatory sequences generally include promoters, ribosome binding sites, and terminator; in eukaryotes, such regulatory sequences generally include promoters, terminators, and in some cases enhancers. Therefore, the term "regulatory sequence" includes all sequences whose existence is the minimum necessary for the expression of the target gene, and may also include other sequences whose existence is advantageous for the expression of the target gene, such as leader sequences.

The term "operably linked" as used herein refers to a situation where the involved sequences are in a relationship that allows them to function in a desired manner. Thus, for example, a regulatory sequence "operably linked" to a coding sequence allows the expression of the coding sequence to be achieved under conditions compatible with the regulatory sequence.

In some embodiments of the fourth aspect, methods well known to those skilled in the art are used to construct a nucleotide sequence that encodes the polypeptide shown in any one of SEQ ID NO: 1 and SEQ ID NO: 4-104 and suitable expression vector for transcription/translation regulatory elements. These methods include in vitro recombinant DNA technology, DNA synthesis technology, in vivo recombination technology, etc. (Sambroook, et al. Molecular Cloning, a Laboratory Manual, cold Spring Harbor Laboratory. New York, 1989). The nucleotide sequence is operably linked to an appropriate promoter in the expression vector to direct mRNA synthesis. Representative examples of these promoters include: *Escherichia coli* lac or trp promoter; lambda phage PL promoter; eukaryotic promoters include CMV immediate early promoter, HSV thymidine kinase promoter, early and late SV40 promoter, Retrovirus LTRs and some other known promoters that can control gene expression in prokaryotic cells or eukaryotic cells or their viruses. The expression vector also includes a ribosome binding site for translation initiation and a transcription terminator. Inserting an enhancer sequence into the vector will enhance its transcription in higher eukaryotic cells. Enhancers are cis-acting factors of DNA expression, usually about 10 to 300 base pairs, acting on promoters to enhance gene transcription. Examples include SV40 enhancers of 100 to 270 base pairs on the late side of the replication initiation point, polyoma enhancers and adenovirus enhancers on the late side of the replication initiation point, and etc.

In addition, the expression vector preferably contains one or more selectable marker genes to provide phenotypic traits for selection of transformed host cells, such as those encoding kanamycin sulfate resistance, ampicillin resistance, and etc.

In the fifth aspect, the present application provides a host cell, which comprises the nucleic acid molecule described in the second aspect or the expression cassette described in the third aspect or the expression vector described in the fourth aspect.

In some embodiments of the fifth aspect, the host cell can express and produce: a polypeptide comprising the amino acid sequence shown in SEQ ID NO:1 or a functional variant thereof, wherein the functional variant has xanthine amide hydrolysis enzyme activity.

In some embodiments of the fifth aspect, wherein the polypeptide has a catalytic site defined as follows in spatial conformation:
The catalytic site comprises amino acid residues H59, H61, K151, H186, H242, and D316 that are close to each other in spatial conformation and refer to SEQ ID NO:1.

In some embodiments of the fifth aspect, the catalytic site further comprises a divalent metal ion.

In some specific embodiments of the fifth aspect, the catalytic site further comprises two divalent metal ions, such as Zn²⁺ and/or Mn²⁺.

In some specific embodiments of the fifth aspect, wherein the polypeptide has a catalytic site and a binding site defined as follows in a spatial conformation:
The catalytic site comprises amino acid residues H59, H61, K151, H186, H242 and D316 that are close to each other in spatial conformation and with reference to SEQ ID NO:1 and 2 divalent metal ions (e.g. Zn²⁺ and/or Mn²⁺).

The binding site comprises amino acid residues I288, A289, P338 and G339 of SEQ ID NO:1 that are close to each other in spatial conformation.

In some embodiments of the fifth aspect, the usable host cell is a cell containing the above-mentioned expression vector, which may be a eukaryotic cell, for example, a yeast cell culture system may be used for the expression of the polypeptide of the present application. The host cell may also be a prokaryotic cell containing the above-mentioned expression vector, for example, may be selected from the genus Escherichia (for example, *Escherichia coli*), *Lactobacillus, Bifidobacterium, Bacteroides, Firmicutes,* and etc.

In some specific embodiments of the fifth aspect, the host cell is a yeast cell or *E*. *coli.*

In some specific embodiments of the fifth aspect, the nucleic acid molecule encoding one or more enzymes of the present application may exist in the host cell in the form of an episomal vector, or may also be integrated into the genome of the host cell.

In some embodiments of any of the above aspects, the isolated nucleic acid is operably linked to a regulatory sequence, which can be recognized by a host cell transformed with the expression vector.

The expression vector can be introduced into the host cell using any technique known in the art, including transformation, transduction, transfection, viral infection, particle bombardment or Ti-mediated gene transfer. Specific methods include calcium phosphate transfection, DEAE-dextran-mediated transfection, lipofection or electroporation (Davis, L., Dibner, M., Battey, I., Basic Methods in Molecular Biology, (1986)). As an example, when the host is a prokaryotic organism such as *Escherichia coli,* competent cells can be harvested after the exponential growth phase and transformed by the CaCl₂ method well known in the art.

In the sixth aspect, the present application provides a pharmaceutical composition or health food, which comprises the polypeptide described in the first aspect, the nucleic acid molecule described in the second aspect, the expression cassette described in the third aspect, and the expression vector described in the fourth aspect, or the host cell described in the fifth aspect and a pharmaceutically acceptable carrier or excipient.

In some embodiments of the sixth aspect, the pharmaceutical composition or health food is used for the prevention, intervention and/or treatment of gout.

In some embodiments of the sixth aspect, the pharmaceutical composition or health food may further comprise one or more of the following: lubricants, such as talc, magnesium stearate and mineral oil; wetting agents; emulsifiers; suspending agents; preservatives, such as benzoic acid, sorbic acid and calcium propionate; sweeteners and/or flavoring agents, etc.

In some embodiments of the sixth aspect, the pharmaceutical composition or health food in the present application can be formulated into tablets, pills, powders, lozenges, elixirs, suspensions, emulsions, solutions, syrups, suppositories or capsules, etc.

In some embodiments of the sixth aspect, any method that can be administered to the intestinal tract can be used to deliver the pharmaceutical composition or health food of the present application, preferably oral administration.

In some specific embodiments of the sixth aspect, the host cells of the fifth invention are prepared into enteric-coated capsules for oral administration.

In some embodiments of the sixth aspect, the drug for therapeutic use can be formulated in the form of a lyophilized preparation or an aqueous solution by mixing a reagent with the required purity with a pharmaceutically acceptable carrier, excipient, etc. and used for storage.

In the seventh aspect, the present application provides the polypeptide described in the first aspect, the nucleic acid molecule described in the second aspect, the expression cassette described in the third aspect, the expression vector described in the fourth aspect, and the host cell described in the fifth aspect, or the pharmaceutical composition or health food in degrading purinesin the sixth aspect.

In some embodiments of the seventh aspect, the degradation of purines occurs in vitro.

In some embodiments of the seventh aspect, the degradation of purines is performed in an anaerobic environment, such as in the intestine.

In the eighth aspect, this application provides the polypeptide described in the first aspect, the nucleic acid molecule described in the second aspect, the expression cassette described in the third aspect, the expression vector described in the fourth aspect, and the host cell described in the fifth aspect, or the pharmaceutical composition or health food in the preparation of a medicament for the prevention, intervention and/or treatment of goutin the sixth aspect.

In the ninth aspect, the present application provides a method for preventing, intervening and/or treating gout, which comprises giving toan individual in needthe polypeptide of the first aspect, the nucleic acid molecule of the second aspect, and the expression cassette of the third aspect, the expression vector according to the fourth aspect, the host cell according to the fifth aspect, or the pharmaceutical composition or health food according to the sixth aspect.

As an exemplary embodiment, in the anaerobic degradation of xanthine, the xanthine amide hydrolase shown in SEQ ID NO: 1 degrades xanthine into 4-ureido-5-carboxyimidazole, thereby reducing the acquisition of purine by human cells,and converting purine into uric acid, thus degrading xanthine in the intestinal tract to prevent, intervene and/or treat gout.

### Examples

The following examples are only illustrative, and are not intended to limit the scope of the embodiments of the present application or the scope of the appended claims.

### Example 1. Preparation of gene clone encoding xanthine amide hydrolase

Using the genome of *Bacillus firmus* (CGMCC 1.2010) as a template, the upstream and downstream primers shown in SEQ ID NOs: 2 and 3 were used to PCR amplify the xanthine amide hydrolase (SEQ ID NO: 1) nucleic acid molecules to obtain the gene sequence encoding xanthine amide hydrolase. Using Gibson's method, the gene sequence encoding xanthine amide hydrolase was inserted into the Sspl restriction site of the HT-1N-TAG-2691 vector (which contains His₆ tag and TEV protease cleavage site) to obtain the expression vector with the gene encoding xanthine amidehydrolase.

### Example 2. Recombinant expression and purification of xanthine amide hydrolase

### 2.1 Recombinant expression

The expression vector containing gene 1 encoding xanthine amide hydrolase constructed in Example 1 was transformed into *E. coli BL21* cells, and spread on LB agar plates containing 50 µg/mL kanamycin sulfate, and incubate overnight at 37°C. Pick a single colony and culture it overnight in 5mL LB liquid medium containing kanamycin sulfate at 220rpm,37°C.Then transfer it to 800mL LB medium for expand cultivation the next day. When the OD 600 value reaches about 0.8, change the temperature to 18°C, and add IPTG at a final concentration of 0.3 mM to induce protein expression. After 16 hours of induction of expression, the cells were collected by centrifugation at 4000xg for 10 min at 4°C. Resuspend the bacterial pellet in 35mL lysis buffer [50mM Tris/HCI, pH 8.0, 1mM phenylmethylsulfonyl fluoride (PMSF), 0.2mg/mL lysozyme, 0.03% Triton X-100 and 1µL DNase I], and frozen and preserved at -80°C.

### 2.2 Purification

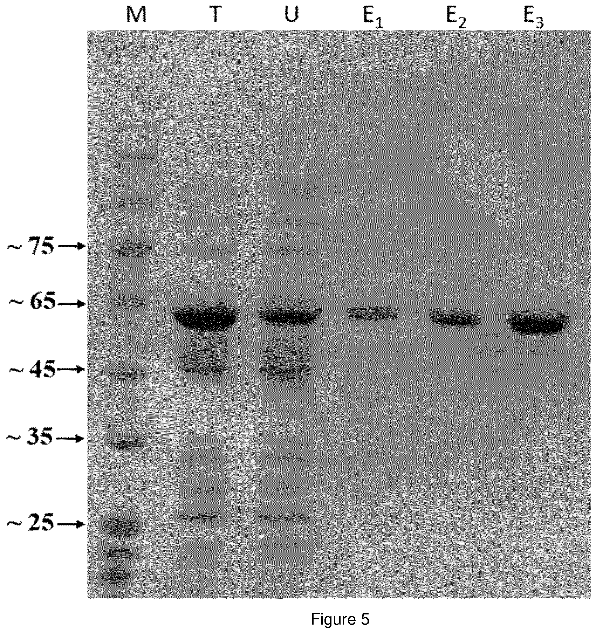

The cells were taken out from -80°C and incubated in a water bath at 25°C for 40 minutes to lyse the cells. Add 6 mL of 6% streptomycin sulfate aqueous solution, mix gently, and centrifuge at 20,000xg for 5 minutes at 4°C. The supernatant was filtered with a 0.22µm filter membrane and bound to a 5mL TALON cobalt column equilibrated with buffer A (20mM Tris-HCl 7.5, 0.2M KCI), and the column was washed with 10 times the column volume of buffer A. Then, the target protein was eluted with 5 column volumes of buffer B containing 150 mM imidazole (20 mM Tris-HCl 7.5, 0.2 M KCI, 150 mM imidazole, 5 mM β-mercaptoethanol). Add ammonium sulfate to the eluted protein solution to 70% saturated precipitated protein, centrifuge at 10,000×g for 10 minutes, discard the supernatant, and use 5mL buffer C (20mM Tris-HCl 7.5, 0.2M KCI, 10 % (V/v) glycerol) to resuspend the precipitate, and a G25 column was used to remove the salt. After removing the salt, the target protein is divided into aliquots, quick-frozen with liquid nitrogen, and stored at -80°C for freezer storage. The final concentration of xanthine amide hydrolase is detected by a spectrophotometer. According to the protein sequence, the extinction coefficient of xanthine amide hydrolase is 55,810M⁻¹ cm⁻¹. On average, about 35mg of protein can be purified per liter. Figure 5 shows the process of expression and purification of xanthine amide hydrolase, and its migration rate on gel electrophoresis conforms to the actual molecular weight (55.7kDa).

### Example 3. Detection of xanthine amide hydrolase activity

Incubate 1 mL of a solution containing 1 mM xanthine, 4 µM xanthine amide hydrolase obtained in Example 2, 0.1 mM Mn²⁺, 200 mM Tris-HCl (pH 7.5) in a water bath at 30°C for 0.5 h to allow the system to fully react, and then add the same volume of methanol solution, mix well, and centrifuged at 14,000xg for 10 minutes, and the protein precipitate was filtered through a 0.22 µm filter membrane, which was recorded as the experimental group. The control group was a reaction without addition of xanthine amide hydrolase and divalent metal ions, and the control group had the same other experimental conditions as the experimental group (Table 1). The experiment was carried out in an anaerobic glove box. All solutions including protein solution, reaction buffer, etc. were deoxygenated through Schlenk line before entering the glove box.

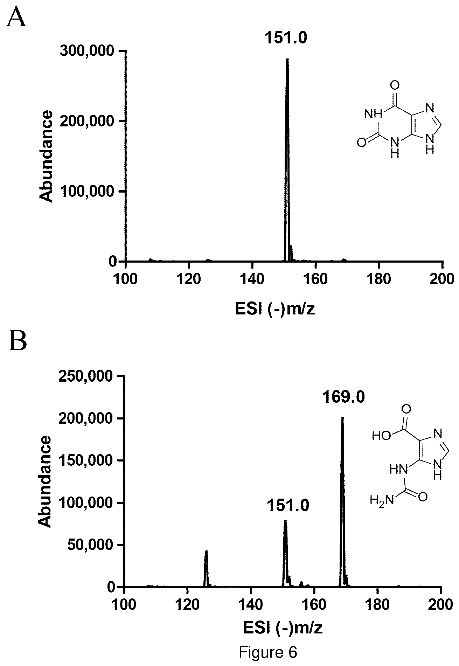

Using LC-MS to detect xanthine amide hydrolase catalyzed the ring opening of xanthine hydrolysis, no product peak was detected in the control group, while the product peak with a molecular weight of 169 Da was detected in the experimental group, which proved that xanthine amide hydrolase can degrade xanthine (Figure 6).

**Table 1**

| | xanthine | xanthine amide hydrolase | Mn²⁺ | Tris-HCl (pH7.5) | ddH₂O |
|---|---|---|---|---|---|
| mother liquor | 1mM | 4µM | 0.1 mM | 200mM | |
| control group | 100µL | 0µL | 0µL | 200µE | 700µL |
| experimental group | 100µL | 100µL | 10µL | 200µL | 590µL |

### Example 4. Identification of the active center of xanthine amide hydrolase

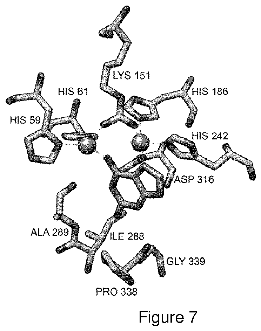

After confirming in vitro that the polypeptide containing the amino acid sequence shown in SEQ ID NO: 1 has xanthine amide hydrolase activity, the PHYRE2 server was used to simulate the structure of the enzyme (Figure 7), showing that it contains fourhistidines, one lysine, one aspartic acid, and two divalent metal ions (Zn²⁺ and/or Mn²⁺)composing a catalytic site. Furthermore, computer software was used to show the anchoring of the substrate xanthine to the simulated xanthine amide hydrolase. The active center also includes one isoleucine, one alanine, one proline and one glycine that are close to each other in spatial conformation,and form the binding site which interacts with the substrate xanthine (Figure 7).

### Example 5. Identification of xanthine amide hydrolaseisozyme

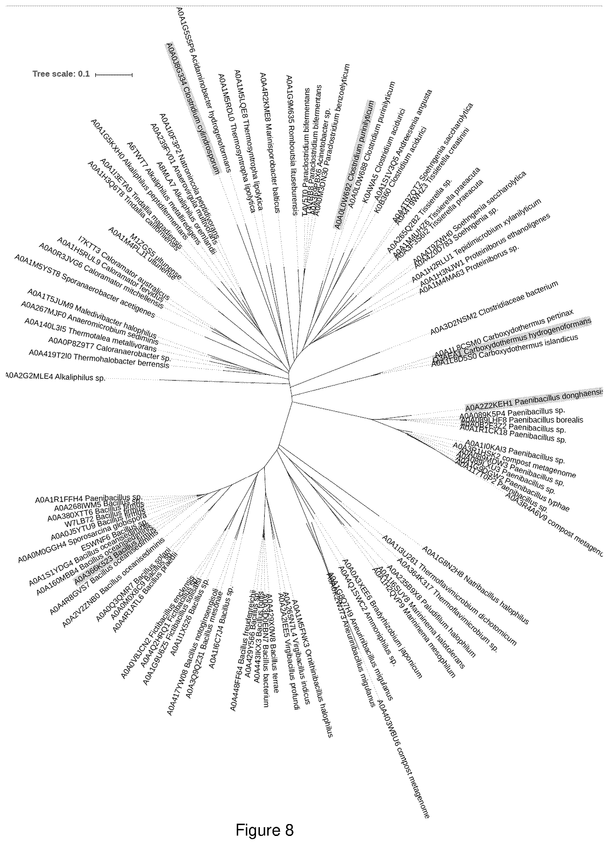

The inventor of the present application further found the sequence of this xanthine amide hydrolase isoenzyme UniRef50 from the Uniprot database (covering 50% sequence identity, and at the same time more than 80% of the xanthine amide hydrolase length of the protein sequence), the above sequences are all in The UniRef50_Q3AEA1 cluster includes a total of 101 protein sequences, and the phylogenetic tree analysis of these 101 proteins from different species (Figure 8). A representative protein is selected from each evolutionary branch, and there are five proteins in total, which are derived from *Bacillus firmus, Clostridium cylindrosporum*DSM 605, *Clostridium purinilyticum, Carbydothermus hydrogenoformans* strain ATCC BAA-161/DSM 6008/Z-2901, and *Paenibacillus donghaensis,* the accession numbers encoded by them are A0A366K523, A0A0J8G334, A0A0L0W692, Q3AEA1, and A0A2Z2KEH1. The sequence comparison results show that the amino acid residues at the above catalytic site and binding site are highly conserved, and all five sequences contain the above amino acid residues, proving that these enzymes are xanthine amide hydrolases (Figure 9). Table 2 shows the accession numbers, strain sources and amino acid sequence numbers of 101 xanthine amide hydrolase enzymes found in the Uniprot database.

**Table 2**

| **Accession number** | **Source of strain** | **SEQ ID NO:** |
|---|---|---|
| A0A0L0W692 | *Clostridium purinilytica* | 4 |
| A0A364K317 | *Thermoflavimicrobium sp.* FBKL4.011 | 5 |
| A0A1I6SUY8 | *Marininema halotolerans* | 6 |
| A0A3D2NSM2 | *Clostridiaceae bacterium* | 7 |
| E5WNF6 | *Bacillus sp.* 2_A_57_CT2 | 8 |
| A0A1R1CK18 | *Paenibacillus sp.* FSLH8-0259 | 9 |
| A0A1I3U261 | *Thermoflavimicrobium dichotomicum* | 10 |
| A0A0V8JCN2 | *Fictibacillus enclensis* | 11 |
| A0A1H2OVP9 | *Marininema mesophilum* | 12 |
| A0A089K5P4 | *Paenibacillus sp.* FSLR5-0912 | 13 |
| A0A1G9DGW2 | *Paenibacillus typhae* | 14 |
| A0A1M4UHZ6 | *Tissierella praeacuta* DSM18095 | 15 |
| A0A1I6C7J4 | *Bacillus sp.* c195 | 16 |
| A0A0A3XEE6 | *Bradyrhizobium japonicum* | 17 |
| A0A380XTT6 | *Bacillus firmus* | 18 |
| A0A1G5S5P6 | *Acidaminobacten hydrogenoformansDSM* 2784 | 19 |
| A0A0P8Z9T7 | *Caloranaerobacter sp*. TR13 | 20 |
| A0A265Q2B2 | *Tissierellasp.* P1 | 21 |
| A0A429Y566 | *Bacillus sp.* 3-2-2 | 22 |
| A0A2Z2KEH1 | *Paenibacillus donghaensis* | 23 |
| K0AWA5 | *Gottschakia aciduric*(ATCC 7906 / DSM 604 / BCRC 14475 / CIP 104303 / NCIMB 10678 / 9a) | 24 |
| A0A443IKX3 | *Bacillus fortis* | 25 |
| A0A2V2ZNB0 | *Bacillus oceantsediminis* | 26 |
| A0A2A2IEE5 | *Virgibacillus profundi* | 27 |
| A0A089MDW3 | *Paenibacillus sp.* FSLR7-0331 | 28 |
| A0A0M0X8C9 | *Bacillus sp.* FJAT-21945 | 29 |
| A0A3F3S6I2 | *Tissierella praeacuta* | 30 |
| A0A1S1YDG4 | *Bacillus oceanisediminis* | 31 |
| A0A1I1X526 | *Bacillus sp.* OV194 | 32 |
| A0A267MJF0 | *Anaeromicrobium sedimints* | 33 |
| A0A1M5RDLO | *Thermosyntropha lipolytica*DSM11003 | 34 |
| A0A1G5KXHO | *Alkaliphilus peptidifermentans*DSM18978 | 35 |
| A0A1G807H9 | *Aneurinibacillus migulanus* | 36 |
| A0A0B2F3Z2 | *Paenibacillussp.*IHB B3415 | 37 |
| A0A4R2KME8 | *Marinisporobacter balticus* | 38 |
| A0A429X0W8 | *Bacillus terrae* | 39 |
| A0A1H3Q6T8 | *Tindallia californiensis* | 40 |
| K0B360 | *Clostridiumaciduric(ATCC* 7906 / DSM 604 /BCRC 14475 / CIP 104303 / NCIMB 10678 / 9a) | 41 |
| A0A1G9M635 | *Romboutsia lituseburensis* DSM797 | 42 |
| T4V5T0 | *Paraclostridium bifermentans* ATCC19299 | 43 |
| A0A4R1ATL6 | *Bacillus praedii* | 44 |
| A0A1L8D5S0 | *Carbydothermus islandicus* | 45 |
| 17KTT3 | *Caloramator australicus* RC3 | 46 |
| T4VRB8 | *Paraclostridium bifermentans* ATCC638 | 47 |
| A0A1H2RLU1 | *Tepidimicrobium xylanilyticum* | 48 |
| A0A0K2WJ73 | *Aneurinibacillus migulanus* | 49 |
| A0A3D0EBN7 | *Bacillus bacterium* | 50 |
| A0A417YW08 | *Bacillus notoginsengisoli* | 51 |
| A0A160MBB4 | *Bacillus oceanisediminis* 2691 | 52 |
| A0A089LXU3 | *Paenibacillus sp.* FSLR7-0273 | 53 |
| MIZGS5 | *[Clostridium] Jultunense Esp* | 54 |
| A0A448FF64 | *Bacillus freudenreichii* | 55 |
| A0A1H5RUL9 | *Caloramator fervidus* | 56 |
| A0A4R8GVS7 | *Bacillus oceanisediminis* | 57 |
| A0A4T9ZWH0 | *Soehngenia saccharolytica* | 58 |
| W7LB72 | *Bacillus frmus* DS1 | 59 |
| A0A140L3I5 | *Thermotalea metallivorans* | 60 |
| A0A1M5FNK3 | *Ornithinibacillus halophilus* | 61 |
| A0A1M5LQES | *Thermosntropha lipolytica* DSM 11003 | 62 |
| A0A4Q2HRQ1 | *Fictibacillus sp.* S7 | 63 |
| A0A3Q9QZ31 | *Bacillus mesonae* | 64 |
| A0A0L0W688 | *Clostridium purinilytica* | 65 |
| A0A1I3ETA9 | *Tindallia magadiensis* | 66 |
| A0A089LHF8 | *Paenibacillus borealis* | 67 |
| A0A0M3DN30 | *Paraclostridium benzoelyticum* | 68 |
| A0A0Q3QMR7 | *Bacillus solani* | 69 |
| A0A419T2I0 | *Thermohalobacter berrensis* | 70 |
| A0A369PBX6 | *Acinetobacter sp.* RIT592 | 71 |
| A0A1T5JUM9 | *Maledivibacter halophils* | 72 |
| A0A4T9WHZ3 | *Tissierella creatinint* | 73 |
| A0A3R1HSK2 | *compost metagenome* | 74 |
| A0A1I0F3P2 | *Natronincola peptidivorans* | 75 |
| A0A0J5YTU9 | *Bacillus firmus* | 76 |
| A0A239FV01 | *Anaerovirgula multivorans* | 77 |
| Q3AEA1 | *Carbydothermus hydrogenoformans, strain* ATCC BAA-161/ DSM 6008 /Z-2901) | 78 |
| A0A1M5YST8 | *Sporanaerobacter acetigenes* DSM13106 | 79 |
| A0A1M4MA63 | *Proteiniborus sp.* DW1 | 80 |
| A0A268IWM5 | *Bacillus sp.* 7894-2 | 81 |
| A0A1M4PLJ1 | *Clostridium]ultunense Esp* | 82 |
| A0A265N7L4 | *Virgibacillus indicus* | 83 |
| A0A1I0KAI3 | *Paenibacillus sp*. NFR01 | 84 |
| A0A1S1V3Q5 | *Andreesenia angusta* | 85 |
| A0A117T0P2 | *Paenibacillus sp.* DMB5 | 86 |
| A0A235B9X8 | *Paludiflum halophtlum* | 87 |
| A0A1H3NJWI | *Proteiniborus ethanoligenes* | 88 |
| A6TWT7 | *Alkaliphilus metalliredigens* (strain QYMF) | 89 |
| A0A1G9U6Z5 | *Fictibacillus solisalsi* | 90 |
| A0A0M0GGH4 | *Sporosarcina globispora* | 91 |
| A0A366K523 | *Bacillus firmus* | 92 |
| A0A1R1FFH4 | *Paenibacillus sp.* FSLR5-0490 | 93 |
| A0A2G2MLE4 | *Alkaliphilus sp.* | 94 |
| A8MLA7 | *Alkaliphilus oremlandii* strain OhILAs | 95 |
| A0A0R3JVG6 | *Caloramator mitchellensis* | 96 |
| A0A0J8G334 | *Clostridium cylindrosporum* DSM605 | 97 |
| A0A4QISWC2 | *Ammoniphilus sp.* CFH90114 | 98 |
| A0A1L8CSM0 | *Carbydothermus pertinax* | 99 |
| A0A4Z0D783 | *Soehngenia sp.* 1933P | 100 |
| A0A4T9ZOT2 | *Soehngenia saccharolytica* | 101 |
| A0AIG8N2H8 | *Natribacillus halophilus* | 102 |
| A0A403WBU6 | *compost metagenome* | 103 |
| A0A3R4A6V9 | *compostmetagenome* | 104 |

### Example 6. Construction of Escherichia coli expressing xanthine amide hydrolase

In this example, *Escherichia coli* capable of expressing xanthine amide hydrolase (SEQ ID NO: 1) was constructed.

The key operation of this example is to integrate the gene encoding xanthine amide hydrolase into the *E*. *coli* genome, for example, one copy of the multi-copy 16sRNA, so that it can be stably expressed, and at the same time, the promoter controlling guanine transporter and guanine deaminase is replaced with the gapA promoter, allowing the gene encoding xanthine amide hydrolase to continuously and stably express.

In this embodiment, it is preferable to replace the promoters encoding guanine transporter and guanine deaminase with the constitutive and continuous and stable expression promoter gapA. The original promoter can usually not promote the gene express when the nitrogen source is sufficientin the environment,unless therelack nitrogen sources or thereis guanine nitrogenin the environment. In order to make the guanine in the food degraded by the xanthine amide hydrolase of the present invention, it is necessary to replace the promoter which controlling guanine transporter and guanine deamination gene expression,and allows them to be expressed at any time, thereby effectively transporting guanine into the above-mentioned engineered *E. coli* cells and degrading it by the sequential action of guanine deaminase and xanthine amide hydrolase, thereby effectively preventing the absorption of guanine in the human intestine and converting it into uric acid.

### Example 7. Escherichia coli expressing xanthine amide hydrolase is used for the treatment of gout

Usingoxonic acid-induced hyper-uric acid rats or uricase knockout transgenic mice as gout animal models, various *Escherichia coli* prepared in Example 6 were made into enteric-coated capsules. Under the same feeding conditions, test whether various *E*. *coli* enteric-coated capsules can reduce the blood uric acid content of gout rats and mouse models.

### Example 8. Escherichia coli expressing xanthine amide hydrolase is used for the treatment of gout

### 8.1 Rat experiment

Select SD male rats (body weight 120g-150g), each group containing 6 rats, under the same feeding conditions, they are divided into experimental group, hyperuricemia group and control group. The specific experimental process is as follows:
1. Animal pre-adaptation: ≥7 days of pre-adaptation process, feeding with ordinary feed and ordinary drinking water.
2. Antibiotic pretreatment: feed with ordinary feed, add 2mg/mL streptomycin + 1mg/mL ampicillin to the drinking water after feeding rats for 3 days, stop drinking water for more than 6 hours, and then give the subsequent gavage treatment.
3. Experimental group: rats were fed with 1% (w/w) adenine feed every day, and treated with 200µL containing 2×10^10 *E. coli* suspension prepared in Example 6 by gavage for two weeks.

Hyperuricemia group: The rats were fed a diet containing 1% (w/w) adenine every day for two weeks.

Control group: rats were fed with ordinary diet daily for two weeks.

After two weeks of feeding, blood was taken from the eye socket, and the serum uric acid content was detected with a uric acid kit.

### 8.2 Mouse experiment

Choose Balb/c mice (8 weeks old), 5 mice in each group, and divide them into experimental group, hyperuricemia group and control group under the same feeding conditions. The specific experimental process is as follows:
1. Animal pre-adaptation: ≥7 days of pre-adaptation process, feeding with ordinary feed and ordinary drinking water.
2. Antibiotic pretreatment: feed with ordinary feed, add 2mg/mL streptomycin and 1mg/mL ampicillin to the drinking water. After feeding the mice for 3 days, stop drinking water for more than 6 hours, then give the follow-up gavage treatment.
3. Experimental group: mice were fed with 0.1% (w/w) adenine feed, and administered with 200 mg/kg potassiumoxonate by gavage every day, and treated with 100 µL containing 2×10^10 *E. coli* suspension prepared in Example 6 by gavage for two weeks.

Hyperuricemia group: mice were fed 0.1% (w/w) adenine feed and 200 mg/kg potassium oxonateby gavage every day for two weeks.

Control group: normal feed was fed every day for two weeks.

After two weeks of feeding, blood was taken from the eye socket, and the serum uric acid content was detected with a uric acid kit.

The exemplary embodiments of the various inventions of the present application are described above. However, without departing from the essence and scope of the present application, those skilled in the art can modify or improve the exemplary embodiments described in the present application. The resulting variants or equivalent solutions also belong to the scope of this application.

## Claims

1. A polypeptide comprising the amino acid sequence shown in SEQ ID NO:1 or a functional variant thereof, wherein the functional variant has xanthine amide hydrolase activity.

2. The polypeptide of claim 1, which has a catalytic site defined as follows in spatial conformation:the catalytic site comprises amino acid residues H59, H61, K151, H186, H242, and D316 that are close to each other in spatial conformation and refer to SEQ ID NO:1.

3. The polypeptide of claim 2, wherein the catalytic site further comprises a divalent metal ion, optionally two divalent metal ions (such as Zn²⁺ and/or Mn²⁺).

4. The polypeptide according to any one of claims 1 to 3, which further comprises a binding site defined as follows in spatial conformation: the binding site comprises amino acid residues I288, A289, P338 and G339 of SEQ ID NO:1 that are close to each other in spatial conformation.

5. The polypeptide of claim 4, wherein the distance between the catalytic site and the binding site is not more than 5 angstroms.

6. The polypeptide of any one of claims 1-5, wherein the functional variant is a natural isozyme of the amino acid sequence shown in SEQ ID NO:1; preferably, the natural isozyme is from: *Bacillus firmus, Compost metagenome, Clostridium purinilyticum, Thermoflavimicrobium sp., Marininemahalotolerans,Clostridiaceae bacterium, Bacillus sp., Paenibacillus sp., Thermoflavimicrobiumdichotomicum, Fictibacillusenclensis, Marininemamesophilum, Paenibacillustyphae, Tissierellapraeacuta, Bradyrhizobium japonicum, Acidaminobacterhydrogenoformans, Caloranaerobacter sp., Tissierella sp., Paenibacillusdonghaensis, Gottschalkiaacidurici, Clostridium acidurici, Bacillus fortis, Bacillus oceanisediminis, Virgibacillus profundi, Anaeromicrobiumsediminis, Thermophilic lipolytica, Alkaliphiluspeptidifermentans, Aneurinibacillusmigulanus, Bacillus bacterium, Marinasporobacterbalticus, Bacillus terrae, Tindalliacaliforniensis, Romboutsialituseburesnis, Paraclostridiumbifermentans, Bacillus praedii, Carbydothermusislandicus, Caloramatoraustralicus, Paraclostridiumbifermentans, Tepidimicrobiumxylanilyticum, Bacillus notoginsengisoli,[Clostridium]ultunenseEsp, Bacillus freudenreichii, Caloramatorfervidus,Soehngeniasaccharolytica, Thermotaleametallivorans, Ornithinibacillushalophilus, Fictibacillussp., Bacillus mesonae, Tindalliamagadiensis, Paenibacillus borealis, Paraclostridiumbenzoelyticum, Bacillus solani, Thermohalobacterberrensis, Acinetobactersp., Maledivibacterhalophilus, Tissierellacreatinini, Natronincolapeptidivorans, Anaerovirgulamultivorans, Carbydothermushydrogenoformans, Sporanaerobacteracetigenes, Proteiniborus sp., Virgibacillus indicus, Andreeseniaangusta, Paludifilumhalophilum, Proteiniborusethanoligenes, Alkaliphilusmetalliredigens, Fictibacillussolisalsi, Sporosarcinaglobispora,Alkaliphilus sp., Alkaliphilusoremlandii, Caloramatormitchellensis, Clostridium cylindrosporum, Ammoniphilus sp., Carbydothermuspertinax, Soehngenia sp.* and *Natribacillushalophilus*;more preferably, the natural isozyme comprises the amino acid sequence shown in any one of SEQ ID NO: 4-104.

7. The polypeptide according to any one of claims 1 to 6, wherein the functional variant is the insertion of one or more amino acids on the basis of the amino acid sequence shown in SEQ ID NO:1 or its natural isozyme, substitution and/or deletion, alternatively, the insertion, substitution and/or deletion does not occur at the catalytic site and/or binding site.

8. A nucleic acid molecule encoding the polypeptide of any one of claims 1-7.

9. An expression cassette comprising the nucleic acid molecule of claim 8.

10. An expression vector comprising the nucleic acid molecule of claim 8 or the expression cassette of claim 9.

11. A host cell comprising the nucleic acid molecule of claim 8, the expression cassette of claim 9, or the expression vector of claim 10.

12. The host cell of claim 11, which is a eukaryotic cell or a prokaryotic cell; preferably, the eukaryotic cell is a yeast cell; preferably, the prokaryotic cell is selected from the group consisting of *Escherichia, Lactobacillus, Bifidobacterium, Bacteroides* and *Firmicutes;* more preferably, the *Escherichia* is *Escherichia coli.*

13. A pharmaceutical composition or health food comprising the polypeptide of any one of claims 1-7, the nucleic acid molecule of claim 8, the expression cassette of claim 9, the expression vector of claim 10, or the host cell of claim 11 or 12 and a pharmaceutically acceptable carrier or excipient.

14. The pharmaceutical composition or health food according to claim 13, which is used for the prevention, intervention and/or treatment of gout.

15. The polypeptide of any one of claims 1-7, the nucleic acid molecule of claim 8, the expression cassette of claim 9, the expression vector of claim 10, the host cell of claim 11 or 12, or the pharmaceutical composition or health food according to claim 13 or 14, have the usage in the degradation of purines.

16. The usage according to claim 15, wherein the degradation of purine occurs in vitro.

17. The polypeptide of any one of claims 1-7, the nucleic acid molecule of claim 8, the expression cassette of claim 9, the expression vector of claim 10, the host cell of claim 11 or 12, or the pharmaceutical composition or health food according to claim 13 or 14,have the usage in the preparation of a medicament for the prevention, intervention and/or treatment of gout.

18. A method for preventing, intervening and/or treating gout, comprising giving toan individual in needthe polypeptide of any one of claims 1-7, the nucleic acid molecule of claim 8, the expression cassette of claim 9,the expression vector of claim 10, the host cell of claim 11 or 12, or the pharmaceutical composition or health food of claim 13 or 14.
